# EUROPEAN PATENT SPECIFICATION

(11) **EP 3 206 594 B1**
(45) Date of publication and mention of the grant of the patent: **25.11.2020**
(21) Application number: 15787402.5
(22) Date of filing: 16.10.2015
(51) Int. Cl.: A61B 17/12

(54) **FIBERING MANDREL**
FASERUNGSDORN
MANDRIN DE FIBRAGE

(30) Priority: 16.10.2014 US 201462064765 P
(43) Date of publication of application: 23.08.2017
(73) Proprietor: Boston Scientific Scimed Inc., Maple Grove, Minnesota 55311 (US)
(72) Inventor: O'BRIEN, John-Alan, County Cork (IE); RYAN, Frank, County Cork (IE); O'SULLIVAN, Conor, County Cork (IE)
(74) Representative: Vossius & Partner Patentanwälte Rechtsanwälte mbB
(86) International application number: PCT/US2015/056041
(87) International publication number: WO 2016/061518

(56) References cited:
- WO-A1-2006/060453
- WO-A2-2008/109228

## Description

### FIELD OF THE INVENTION

This application relates to the field of medical device manufacture. More particularly, the application is related to devices and methods for the manufacture of fibered occlusive coils.

### BACKGROUND

Therapeutic embolization or occlusion of blood vessels may be used to treat a variety of vascular and non-vascular conditions including cerebral and peripheral aneurysms, ateriovenous malformation, uterine fibroids and various tumors or other peripheral vascular disorders or dysfunctions. One commonly used agent for embolizing blood vessels is the embolic coil, a permanently implanted coiled structure which, when implanted into a blood vessel, occludes the vessel by causing thrombosis where it is deployed. Embolic coils may have different lengths and/or cross-sectional diameters, in order to fit into and occlude vascular structures of varying sizes. Embolic coils frequently include one or more thrombogenic materials, most often polymer fibers, which are most often inserted (singly or as bundles) between individual windings of the coil. The quantity and position of these fibers may significantly affect the ease with which a given embolic coil is delivered to a patient, as well as its effectiveness as an occlusive agent, so consistent positioning of these fibers is generally desirable.

To occlude a vascular structure, one or more such coils is delivered, for example via a microcatheter, to a site where occlusion is desired. Within the catheter, the coil is held in an elongate, compressed configuration, but assumes a complex three-dimensional shape when discharged into the vasculature. The coil typically includes an atraumatic feature at its distal-most terminus, such as a rounded head, and includes a detachable junction to mechanically connect the coil to a pushrod (also referred to as a "delivery wire;" the terms are used interchangeably) within the deployment microcatheter.

WO 2008/109228 A2 describes an embolic coil delivery system that includes a catheter and an embolic coil disposed within the catheter, the embolic coil including a coil wire and a fiber bundle.

WO 2006/060453 A1 describes embolic coils as well as related methods, devices and compositions.

### SUMMARY OF THE INVENTION

The invention is defined in independent claims 1 and 12. Certain optional features of the invention are defined in the dependent claims. The present invention, in its various aspects, meets an ongoing need in the field for the efficient and rapid manufacture of fibered embolic coils.

In one aspect, the present invention relates to a mandrel for attaching fibers to an embolic coil. The mandrel includes opposing first and second ends, and a slot extending between them. At one end, a retaining element engages a first (or distal) end of the embolic coil, while an anchoring mechanism engages a second (or proximal) end of the coil. The mandrel includes a plurality of castellations extending along the slot between the first and second ends. In the various embodiments of the mandrel, the retaining element may be shaped or configured to engage an atraumatic ball tip of the coil. The mandrel may include one or more of a cutting groove on the side of the mandrel opposite the slot and a marking referencing at least one of the castellations (e.g. to indicate the placement of a fiber or bundle of fibers). The anchoring mechanism and/or the retaining element may be slidably disposed along the mandrel, to permit the assembly of coils of different lengths.

In another aspect, the present invention relates to a method of manufacturing an embolic coil which utilizes a mandrel such as those described above. The method involves inserting an embolic coil into the slot, and optionally sliding the anchoring mechanism and/or the retaining element into place along the mandrel and/or engaging the retaining element and anchoring mechanism with the coil to secure it in place. The method also includes wrapping at least one fiber around the mandrel and through at least one castellation, thereby inserting the fiber between adjacent coil windings, and severing the fiber. In some cases, the castellations include a ramp feature where the castellation joins the mandrel, to improve seating of fibers between windings of the coil. In cases where the mandrel includes a cutting groove opposite the slot, the method also includes sliding a cutting instrument (such as a razor blade) through the cutting groove to sever windings of the fiber or fibers, thereby cutting the fibers within the coil to a predetermined length.

### DRAWINGS

Aspects of the invention are described below with reference to the following drawings in which like numerals reference like elements, and wherein:

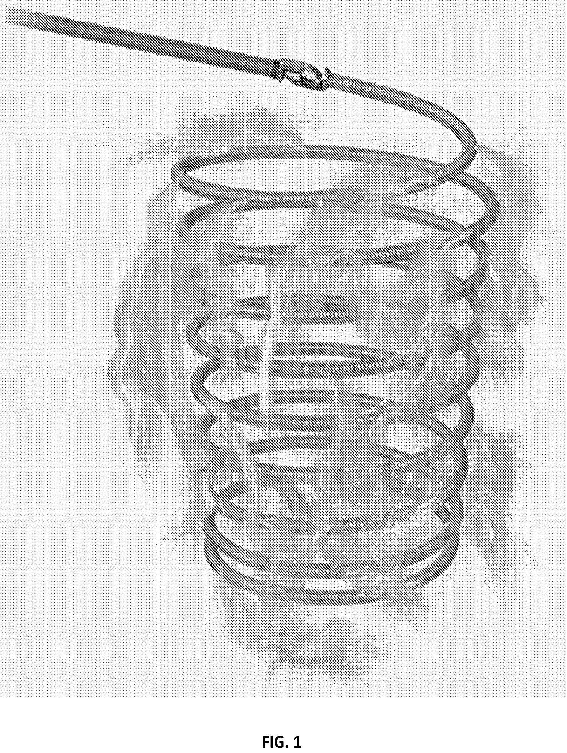

FIG. 1 includes a schematic perspective drawing of exemplary fibered embolic coil mechanically joined to a delivery wire.
FIG. 2 includes a photograph of an exemplary coil fibering mandrel
FIG. 3A-B includes a photograph of a slot sized to accommodate an embolic coil for fibering. FIG. 3C shows a retaining element which engages a distal atraumatic tip of the embolic coil and holds it in a fixed longitudinal position within the slot.
FIG. 4A-C depict anchoring structures to which proximal coil ends may be attached. The retaining element, slot and anchoring structure all enable a shaped secondary coil to be straightened out for fibering, without damaging the secondary shape of the product.
FIG. 5A depicts fibering castellations adjoining the coil slot and their spatial relationship to the distal retaining element. FIG. 5B-C depicts the castellations and their relationship to the base of the mandrel. FIG. 5D shows a cutting groove opposite the slot, and FIG. 5E-F shows fibers wrapped around the mandrel and the coil and then cut by sliding a blade through the cutting groove as part of the fibering process. FIG. 5G shows a side groove for cutting fibers to length.
FIG. 6A-B depicts a ramp feature which aids in fiber placement in certain embodiments of the invention.

Unless otherwise provided in the following specification, the drawings are not necessarily to scale, with emphasis being placed on illustration of the principles of the invention.

### DETAILED DESCRIPTION OF THE PREFERRED EMBODIMENTS

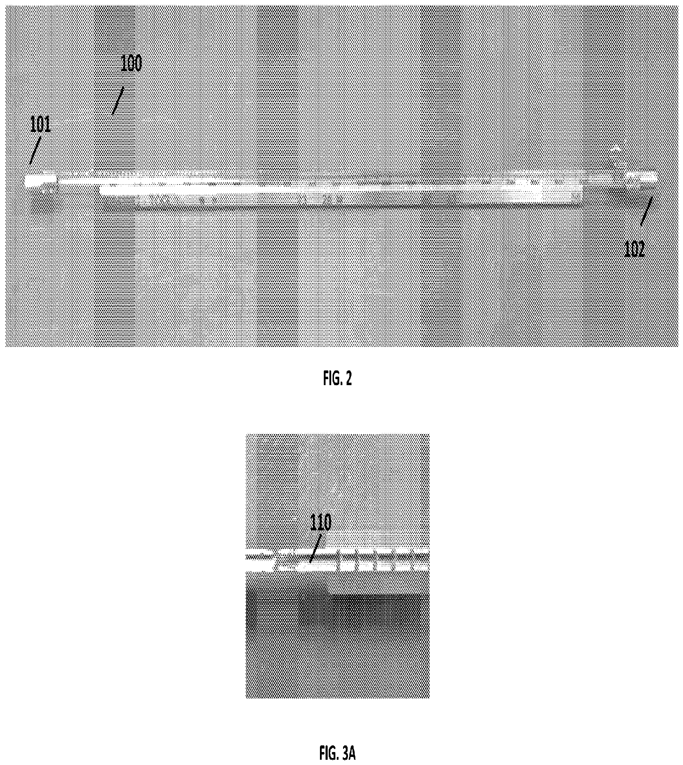

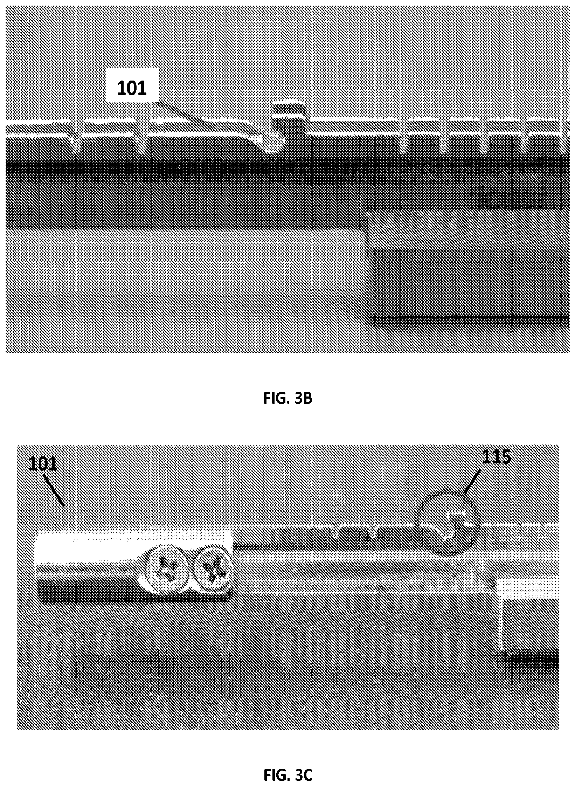

Referring to FIG. 2, an exemplary fibering mandrel 100 is generally elongate, with first and second ends 101, 102. A slot 110, as shown in FIG. 3A-B, extends along the mandrel 100 between the first and second ends. Near the first end 101, a retaining element 115 is positioned to engage with a distal end of the coil when it is laid into the slot 110. While the retaining element may have any suitable shape, in certain embodiments, such as the one shown in Fig. 3C, the retaining element defines a rounded void to accommodate the rounded atraumatic distal tip of an embolic coil.

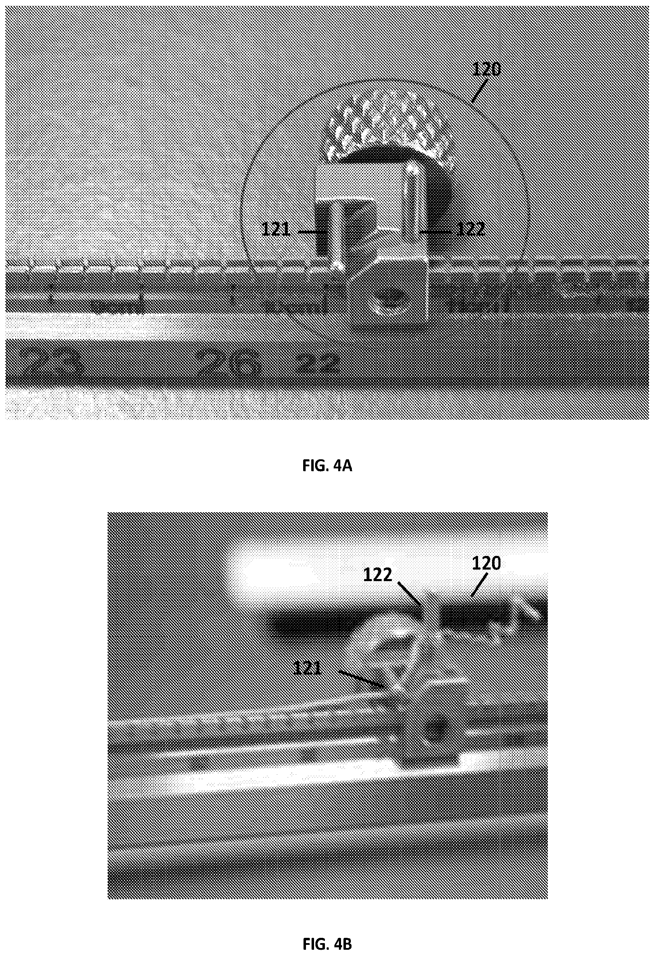

Nearer the second end of the mandrel 100 is an anchoring mechanism 120 for the proximal end of the embolic coil, as shown in Fig. 4A-C. In some embodiments, as shown in Fig. 4A-B, the anchoring mechanism 120 is slidably positioned along the mandrel in order to facilitate the fibering of coils of different lengths, and is lockable (in this case via a thumbscrew) to prevent changes in the position of the coil or the tension applied to it during use. In the exemplary embodiments shown in Fig. 4A-B, the anchoring mechanism 120 includes first and second angled posts 121, 122. The first post 120 is preferably positioned so as to minimally displace the coil relative to the slot 110. To anchor a coil to the anchoring element 120, the coil is threaded beneath the first post 121 and then wrapped one or more times about the second post 122, thereby securing it in place.

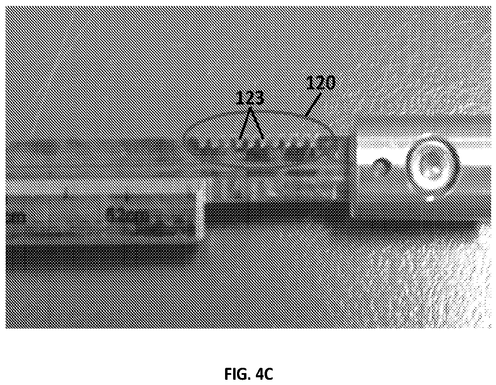

In some cases, as shown in Fig. 4C, the anchoring mechanism 120 does not include any moving parts, and is instead simply one or more grooves 123 angled relative to the slot 110; in use, the proximal end of the coil is wound about the mandrel and through the one or more grooves 123, thereby securing it for the fibering procedures

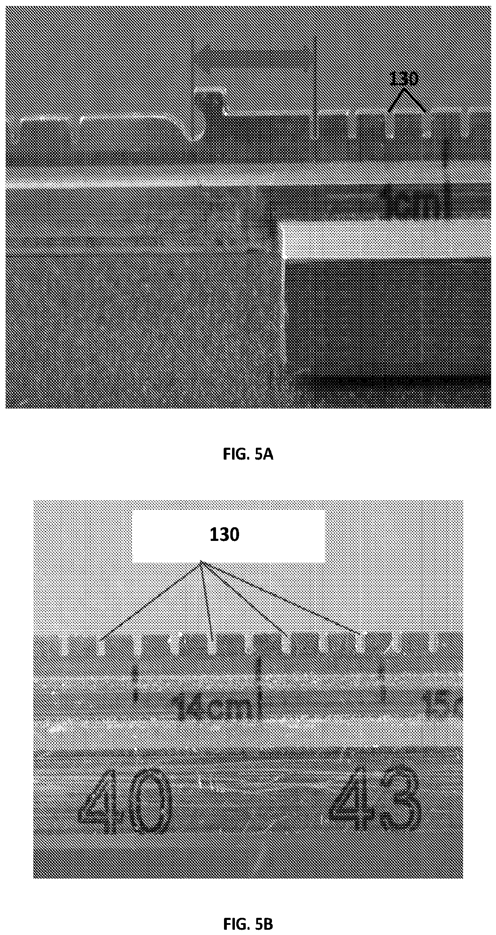

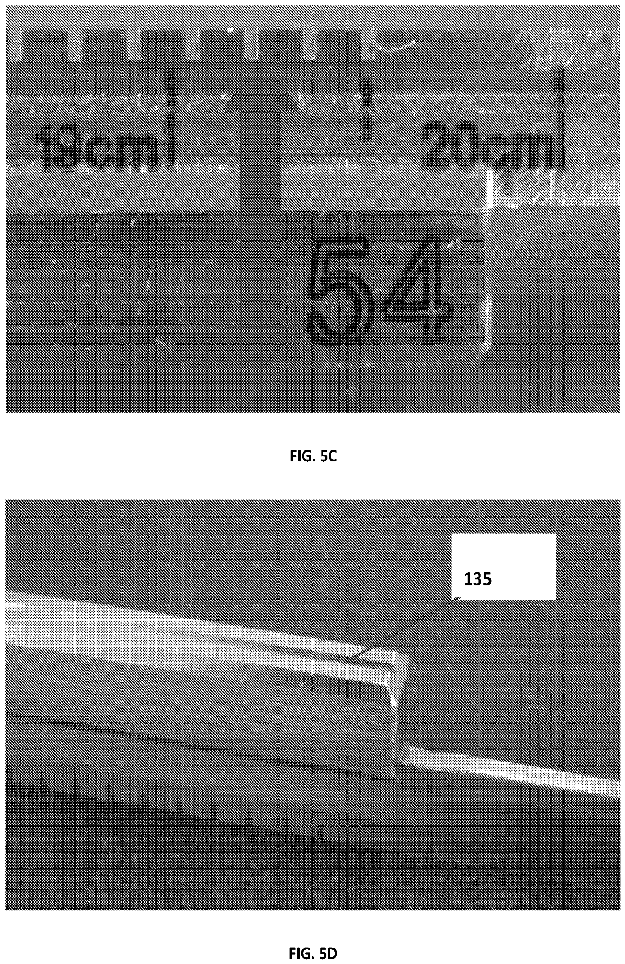

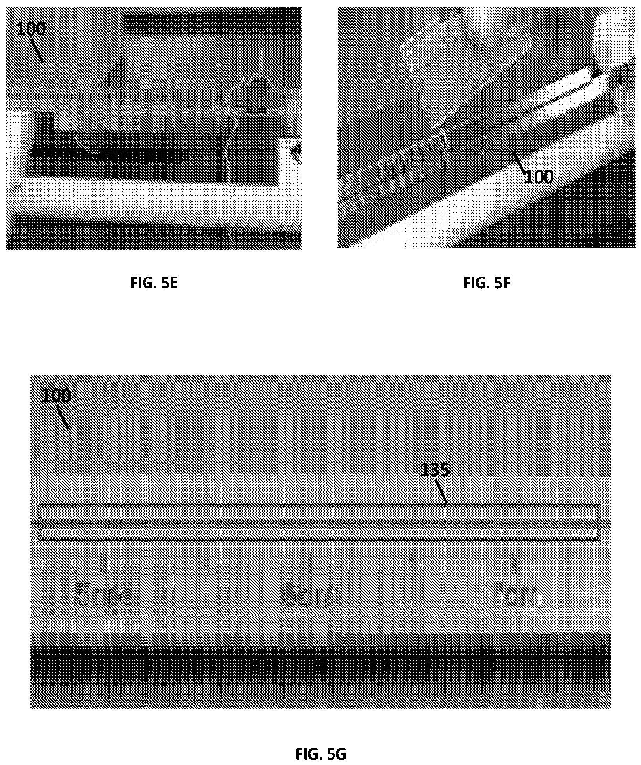

Turning to Fig. 5, the mandrel 100 includes a plurality of castellations 130 extending between the first and second ends alongside the slot 110. The castellations are generally sized and spaced to permit the insertion of fibers, either singly or in bundles, to be inserted between individual windings of the embolic coil, thereby securing them to the coil. The castellations 130 are, in preferred embodiments, spaced at fixed distances from the first and second ends of the mandrel 100 and/or from each of the retaining element 115 and the anchoring element 120, as illustrated in Fig. 5A. The mandrel 100 also preferably includes one or more features to assist in the accurate placement of fibers or bundles of fibers, such as distance markings or markings to indicate the number of wraps, both of which are shown in Fig. 5B. As the fibers are meant to be wrapped around the mandrel 100, its physical dimensions, including width (as shown in Fig. 5C), thickness, or diameter, govern the final length of the fibers attached to the embolic coil. The mandrel also includes a cutting groove 135 (see Figs. 5D, 5G) through which a blade can be moved to cut fibers wrapped around the mandrel 100. The cutting groove 135 can be on the mandrel 100 opposite the slot 110 (see Fig. 5D) and/or on each side of the mandrel 135 (see Fig. 5G), to facilitate cutting the fiber or fibers to a specific length.

In use, as shown in Fig. 5D-F the mandrel is placed horizontally on a workbench with the slot 110 facing up and an embolic coil is placed within the slot 110 and secured to each of the retaining element 115 and the anchoring mechanism 120, to hold it in place during fibering. One or more polymer fibers are inserted into the first castellation 130 of the mandrel 100 (generally relative to the first end of the mandrel), and are then wrapped around the mandrel 100 and through a second, marked castellation 130. Thereafter, the fiber or fibers are wrapped sequentially toward the second end of the mandrel 100 and through a specified series of marked castellations 130. When the fiber or fibers are fully wrapped about the mandrel (i.e. when the fibers have been inserted into each castellation required by the applicable product specification), the fibers are preferably secured in place to prevent slippage, then the mandrel is turned over to expose the cutting groove 135, and the user slides a sharp edge of a blade through the cutting groove 135, thereby severing the looped fiber or fibers into a discrete series of individual fibers or fiber bundles.

Fibering mandrels according to the invention advantageously permit efficient and accurate placement of fibers within embolic coils, facilitating the speed of manufacture relative to fibering done without the aid of a mandrel. In addition, adjustable features such as the anchoring mechanism 120 permit coils of different lengths to be fibered using a single mandrel design. Similarly, the inclusion of markings facilitates the precise placement of fibers or bundles.

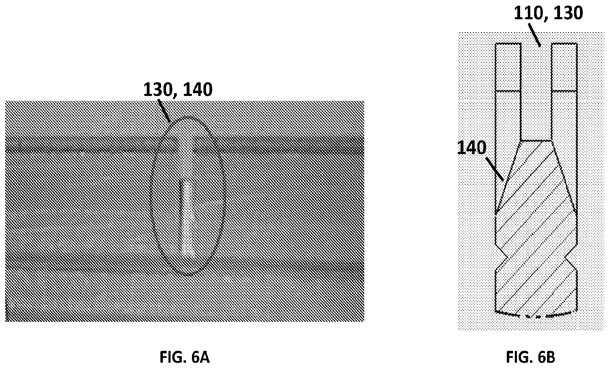

In some embodiments, the mandrel 100 includes structural features that further facilitate the wrapping of fibers. For instance, Fig. 6 depicts ramp elements 140 below a castellation 130, to permit the fiber or fibers to form a more acute angle than can be otherwise attained. This prevents the deflection of the mandrel under the tension applied by the fibers, and allows the fiber or fibers to lie more closely to the interior of the wire of the coil when inserted.

The phrase "and/or," as used herein should be understood to mean "either or both" of the elements so conjoined, i.e., elements that are conjunctively present in some cases and disjunctively present in other cases. Other elements may optionally be present other than the elements specifically identified by the "and/or" clause, whether related or unrelated to those elements specifically identified unless clearly indicated to the contrary. Thus, as a non-limiting example, a reference to "A and/or B," when used in conjunction with open-ended language such as "comprising" can refer, in one embodiment, to A without B (optionally including elements other than B); in another embodiment, to B without A (optionally including elements other than A); in yet another embodiment, to both A and B (optionally including other elements); etc.

The term "consists essentially of" means excluding other materials that contribute to function, unless otherwise defined herein. Nonetheless, such other materials may be present, collectively or individually, in trace amounts.

As used in this specification, the term "substantially" or "approximately" means plus or minus 10% (e.g., by weight or by volume), and in some embodiments, plus or minus 5%. Reference throughout this specification to "one example," "an example," "one embodiment," or "an embodiment" means that a particular feature, structure, or characteristic described in connection with the example is included in at least one example of the present technology. Thus, the occurrences of the phrases "in one example," "in an example," "one embodiment," or "an embodiment" in various places throughout this specification are not necessarily all referring to the same example. Furthermore, the particular features, structures, routines, steps, or characteristics may be combined in any suitable manner in one or more examples of the technology. The headings provided herein are for convenience only and are not intended to limit or interpret the scope or meaning of the claimed technology.

Certain embodiments of the present invention have described above. It is, however, expressly noted that the present invention is not limited to those embodiments, but rather the intention is that additions and modifications to what was expressly described herein are also included within the scope of the invention. Moreover, it is to be understood that the features of the various embodiments described herein were not mutually exclusive and can exist in various combinations and permutations, even if such combinations or permutations were not made express herein, without departing from the scope of the invention. In fact, variations, modifications, and other implementations of what was described herein will occur to those of ordinary skill in the art without departing from the scope of the invention. As such, the invention is not to be defined only by the preceding illustrative description.

## Claims

1. A mandrel (100) for attaching fibers to an embolic coil, the mandrel comprising:
a first end (101) and a second end (102) opposite the first end (101);
a slot (110) extending from the first end (101) to the second end (102) and sized to accommodate the embolic coil, **characterized by**
a retaining element (115) configured to engage a distal end of the embolic coil;
an anchoring mechanism (120) configured to engage a proximal end of the embolic coil opposite the distal end; and
a plurality of castellations (130) extending along the slot between the first and second ends, wherein the castellations (130) are generally sized and spaced to permit the insertion of fibers, either singly or in bundles, to be inserted between individual windings of the embolic coil, thereby securing them to the coil.

2. The mandrel (100) of claim 1, wherein the retaining element (115) is configured to engage an atraumatic ball tip of the embolic coil.

3. The mandrel (100) of any one of claims 1-2, further comprising a cutting groove (135) opposite the slot (110).

4. The mandrel (100) of any one of claims 1-3, further comprising at least one marking referencing at least one castellation (130).

5. The mandrel (100) of any one of claims 1-4, wherein the anchoring mechanism (120) is slidably disposed along the mandrel (100).

6. The mandrel (100) of any one of claims 1-5, wherein each castellation (130) includes a ramp (140).

7. The mandrel (100) of claim 1, wherein at least one of the retaining element (115) and the anchoring mechanism (120) is slidably disposed along the mandrel (100).

8. The mandrel (100) of claim 7, wherein the retaining element (115) is configured to engage an atraumatic ball tip of the embolic coil,

9. The mandrel (100) of any one of claims 7-8, further comprising a cutting groove (135) opposite the slot (110).

10. The mandrel (100) of any one of claims 7-9, further comprising at least one marking referencing at least one castellation (130).

11. The mandrel (100) of any one of claims 7-10, wherein each castellation (130) includes a ramp (140).

12. A method of manufacturing an embolic coil using the mandrel (100) of any one of claims 1-6, the method comprising the steps of:
inserting an embolic coil within the slot (110);
wrapping at least one fiber around the mandrel (100) and through at least one castellation (130), thereby inserting the fiber between adjacent windings of the coil; and
severing the at least one fiber.

13. The method of claim 12, wherein the anchoring mechanism (120) includes first and second angled posts (121, 122), and the method includes winding at least a portion of the embolic coil around one of the first and second posts (121, 122), thereby engaging the anchoring mechanism (120) and the embolic coil.

14. The method of claim 12 using the mandrel (100) of any one of claims 7 to 11 instead of using the mandrel (100) of any one of claims 1-6, the method comprising the steps of:
sliding at least one of the retaining element (115) and the anchoring mechanism (120) along the mandrel (100); and
engaging the retaining element (115) and the anchoring mechanism (120) with the coil, thereby securing the coil.

15. The method of claim 14, wherein the anchoring mechanism (120) includes first and second angled posts (121, 122), and the method includes winding at least a portion of the embolic coil around one of the first and second posts (121, 122), thereby engaging the anchoring mechanism (120) and the embolic coil.

## Patentansprüche

1. Dorn (100) zum Befestigen von Fasern an einer Emboliespule, wobei der Dorn aufweist:
ein erstes Ende (101) und ein dem ersten Ende (101) gegenüberliegendes zweites Ende (102);
einen Schlitz (110), der sich vom ersten Ende (101) zum zweiten Ende (102) erstreckt und dafür dimensioniert ist, die Emboliespule aufzunehmen, **gekennzeichnet durch**
ein Halteelement (115), das dafür konfiguriert ist, mit einem distalen Ende der Emboliespule in Eingriff zu kommen;
einen Verankerungsmechanismus (120), der dafür konfiguriert ist, mit einem dem distalen Ende gegenüberliegenden proximalen Ende der Emboliespule in Eingriff zu kommen; und
mehrere Kastellierungen (130), die sich entlang des Schlitzes zwischen dem ersten und dem zweiten Ende erstrecken, wobei die Kastellierungen (130) allgemein derart dimensioniert und beabstandet sind, dass es ermöglicht wird, Fasern entweder einzeln oder in Bündeln zwischen einzelnen Wicklungen der Emboliespule einzuführen, um sie an der Spule zu befestigen.

2. Dorn (100) nach Anspruch 1, wobei das Halteelement (115) dafür konfiguriert ist, mit einer atraumatischen Kugelspitze der Emboliespule in Eingriff zu kommen.

3. Dorn (100) nach Anspruch 1 oder 2, ferner mit einer Schneidnut (135) gegenüber dem Schlitz (110).

4. Dorn (100) nach einem der Ansprüche 1 bis 3, ferner mit mindestens einer Markierung, die auf mindestens eine Kastellierung (130) Bezug nimmt.

5. Dorn (100) nach einem der Ansprüche 1 bis 4, wobei der Verankerungsmechanismus (120) entlang des Dorns (100) gleitend angeordnet ist.

6. Dorn (100) nach einem der Ansprüche 1 bis 5, wobei jede Kastellierung (130) eine Rampe (140) aufweist.

7. Dorn (100) nach Anspruch 1, wobei mindestens eine Komponente unter dem Halteelement (115) und dem Verankerungsmechanismus (120) entlang des Dorns (100) gleitend angeordnet ist.

8. Dorn (100) nach Anspruch 7, wobei das Halteelement (115) dafür konfiguriert ist, mit einer atraumatischen Kugelspitze der Emboliespule in Eingriff zu kommen.

9. Dorn (100) nach einem der Ansprüche 7 bis 8, ferner mit einer Schneidnut (135) gegenüber dem Schlitz (110).

10. Dorn (100) nach einem der Ansprüche 7 bis 9, ferner mit mindestens einer Markierung, die auf mindestens eine Kastellierung (130) Bezug nimmt.

11. Dorn (100) nach einem der Ansprüche 7 bis 10, wobei jede Kastellierung (130) eine Rampe (140) aufweist.

12. Verfahren zum Herstellen einer Emboliespule unter Verwendung des Dorns (100) nach einem der Ansprüche 1 bis 6, wobei das Verfahren die Schritte aufweist:
Einsetzen einer Emboliespule in den Schlitz (110);
Wickeln mindestens einer Faser um den Dorn (100) und durch mindestens eine Kastellierung (130), wodurch die Faser zwischen benachbarten Wicklungen der Spule eingeführt wird; und
Durchtrennen der mindestens einen Faser.

13. Verfahren nach Anspruch 12, wobei der Verankerungsmechanismus (120) ein erstes und ein zweites abgewinkeltes Stabelement (121, 122) aufweist, und wobei das Verfahren das Wickeln mindestens eines Teils der Emboliespule um das erste oder das zweite Stabelement (121, 122) aufweist, wodurch der Verankerungsmechanismus (120) und die Emboliespule miteinander in Eingriff gebracht werden.

14. Verfahren nach Anspruch 12 unter Verwendung des Dorns (100) nach einem der Ansprüche 7 bis 11 anstelle der Verwendung des Dorns (100) nach einem der Ansprüche 1 bis 6, wobei das Verfahren die Schritte aufweist:
Verschieben mindestens einer Komponente unter dem Halteelement (115) und dem Verankerungsmechanismus (120) entlang des Dorns (100); und
Ineingriffbringen des Halteelements (115) und des Verankerungsmechanismus (120) mit der Spule, um die Spule zu befestigen.

15. Verfahren nach Anspruch 14, wobei der Verankerungsmechanismus (120) ein erstes und ein zweites abgewinkeltes Stabelement (121, 122) aufweist, und wobei das Verfahren das Wickeln mindestens eines Teils der Emboliespule um das erste oder das zweite Stabelement (121, 122) aufweist, um den Verankerungsmechanismus (120) mit der Emboliespule in Eingriff zu bringen.

## Revendications

1. Mandrin (100) pour fixer des fibres à une spirale embolique, le mandrin comprenant :
une première extrémité (101) et une seconde extrémité (102) opposée à la première extrémité (101) ;
une fente (110) s'étendant de la première extrémité (101) à la seconde extrémité (102) et dimensionnée pour loger la spirale embolique, **caractérisé par** :
un élément de retenue (115) configuré pour mettre en prise une extrémité distale de la spirale embolique ;
un mécanisme d'ancrage (120) configuré pour mettre en prise une extrémité proximale de la spirale embolique opposée à l'extrémité distale ; et
une pluralité de crénelures (130) s'étendant le long de la fente entre les première et seconde extrémités, dans lequel les crénelures (130) sont généralement dimensionnées et espacées pour permettre l'insertion de fibres, soit individuellement ou en paquets, à insérer entre des enroulements individuels de la spirale embolique, les fixant ainsi sur la spirale.

2. Mandrin (100) selon la revendication 1, dans lequel l'élément de retenue (115) est configuré pour mettre en prise une pointe sphérique atraumatique de la spirale embolique.

3. Mandrin (100) selon l'une quelconque des revendications 1 à 2, comprenant en outre une rainure de coupe (135) opposée à la fente (110).

4. Mandrin (100) selon l'une quelconque des revendications 1 à 3, comprenant en outre au moins un marquage référençant au moins une crénelure (130).

5. Mandrin (100) selon l'une quelconque des revendications 1 à 4, dans lequel le mécanisme d'ancrage (120) est disposé, de manière coulissante, le long du mandrin (100).

6. Mandrin (100) selon l'une quelconque des revendications 1 à 5, dans lequel chaque crénelure (130) comprend une rampe (140).

7. Mandrin (100) selon la revendication 1, dans lequel au moins l'un parmi l'élément de retenue (115) et le mécanisme d'ancrage (120) est disposé, de manière coulissante, le long du mandrin (100).

8. Mandrin (100) selon la revendication 7, dans lequel l'élément de retenue (115) est configuré pour mettre en prise une pointe sphérique atraumatique de la spirale embolique.

9. Mandrin (100) selon l'une quelconque des revendications 7 à 8, comprenant en outre une rainure de coupe (135) opposée à la fente (110).

10. Mandrin (100) selon l'une quelconque des revendications 7 à 9, comprenant en outre au moins un marquage référençant au moins une crénelure (130).

11. Mandrin (100) selon l'une quelconque des revendications 7 à 10, dans lequel chaque crénelure (130) comprend une rampe (140).

12. Procédé pour fabriquer une spirale embolique à l'aide du mandrin (100) selon l'une quelconque des revendications 1 à 6, le procédé comprenant les étapes suivantes :
insérer une spirale embolique dans la fente (110) ;
enrouler au moins une fibre autour du mandrin (100) et à travers au moins une crénelure (130), insérant ainsi la fibre entre les enroulements adjacents de la spirale ; et
couper la au moins une fibre.

13. Procédé selon la revendication 12, dans lequel le mécanisme d'ancrage (120) comprend des premier et second montants coudés (121, 122) et le procédé comprend l'étape pour enrouler au moins une partie de la spirale embolique autour de l'un des premier et second montants (121, 122), mettant ainsi en prise le mécanisme d'ancrage (120) et la spirale embolique.

14. Procédé selon la revendication 12, utilisant le mandrin (100) selon l'une quelconque des revendications 7 à 11 au lieu d'utiliser le mandrin (100) selon l'une quelconque des revendications 1 à 6, le procédé comprenant les étapes suivantes :
faire coulisser au moins l'un parmi l'élément de retenue (115) et le mécanisme d'ancrage (120) le long du mandrin (100) ; et
mettre en prise l'élément de retenue (115) et le mécanisme d'ancrage (120) avec la spirale, fixant ainsi la spirale.

15. Procédé selon la revendication 14, dans lequel le mécanisme d'ancrage (120) comprend des premier et second montants coudés (121, 122) et le procédé comprend l'étape pour enrouler au moins une partie de la spirale embolique autour de l'un des premier et second montants (121, 122), mettant ainsi en prise le mécanisme d'ancrage (120) et la spirale embolique.
